# EUROPEAN PATENT SPECIFICATION

(11) **EP 3 071 177 B1**
(45) Date of publication and mention of the grant of the patent: **10.05.2017**
(21) Application number: 14800064.9
(22) Date of filing: 20.11.2014
(51) Int. Cl.: C07K 14/315, A61K 8/64, A61K 8/11, A61Q 19/02

(54) **TARGETED CAPSULES FOR THE DELIVERY OF SKIN WHITENING AGENTS IN THE SKIN**
GEZIELTE KAPSELN ZUR ABGABE VON HAUTAUFHELLUNGSMITTELN IN DIE HAUT
CAPSULES CIBLÉES POUR L'ADMINISTRATION D'AGENTS CUTANÉS BLANCHISSANTS DANS LA PEAU

(30) Priority: 20.11.2013 EP 13382467
(43) Date of publication of application: 28.09.2016
(60) Divisional application: 16178588.6
(73) Proprietor: Infinitec Activos S.L., 08170 Montornés del Vallès, Barcelona (ES)
(72) Inventor: MOURELLE MANCINI, Marisabel, E-08028 Barcelona (ES); CARCELLER MARGELI, Magdalena, E-08907 Hospitalet de Llobregat (ES)
(74) Representative: ABG Patentes, S.L.
(86) International application number: PCT/EP2014/075120
(87) International publication number: WO 2015/075116

(56) References cited:
- WO-A1-2012/013136
- WO-A2-2009/118191
- WO-A2-2011/097384
- FR-A1- 2 911 280
- US-A1- 2006 216 254
- US-A1- 2013 203 670
- BEDNAREK M A ET AL: "Analogs of [alpha]-melanocyte stimulating hormone with high agonist potency and selectivity at human melanocortin receptor 1b: the role of Trp<9> in molecular recognition", BIOPOLYMERS JOHN WILEY & SONS INC. USA, vol. 89, no. 5, May 2008 (2008-05), pages 401-408, XP055112702, ISSN: 0006-3525
- BEDNAREK M A ET AL: "Cyclic analogs of alpha-melanocyte-stimulating hormone (alphaMSH) with high agonist potency and selectivity at human melanocortin receptor 1b", PEPTIDES, ELSEVIER, AMSTERDAM, NL, vol. 29, no. 6, 1 June 2008 (2008-06-01), pages 1010-1017, XP022646987, ISSN: 0196-9781, DOI: 10.1016/J.PEPTIDES.2008.02.008 [retrieved on 2008-02-19]
- SCHIOTH HELGI B ET AL: "SUBTYPE SELECTIVE BINDING PROPERTIES OF SUBSTITUTED LINEAR MELANOCYTE STIMULATING HORMONE ANALOGUES", NEUROPEPTIDES, CHURCHILL LIVINGSTONE, GB, vol. 36, no. 6, 1 December 2002 (2002-12-01), pages 427-434, XP009076076, ISSN: 0143-4179, DOI: 10.1016/S0143-4179(02)00133-6
- SCHIOTH HELGI B ET AL: "Selective properties of C- and N-terminals and core residues of the melanocyte-stimulating hormone on binding to the human melanocortin receptor subtypes", EUROPEAN JOURNAL OF PHARMACOLOGY, vol. 349, no. 2-3, 22 May 1998 (1998-05-22), pages 359-366, XP002723838, ISSN: 0014-2999

### SEQUENCE LISTING

<110> INFINITEC ACTIVOS, S.L.
   PEPTIDEPHARMA NOVA, S.L.
<120> TARGETED CAPSULES FOR THE DELIVER OF SKIN WHITENING AGENTS IN THE SKIN
<130> P10071PC00
<150> EP13382467.2
   <151> 2013-11-20
<160> 4
<170> PatentIn version 3.5
<210> 1
   <211> 13
   <212> PRT
   <213> Homo sapiens
<220>
   <221> MOD_RES
   <222> (1)..(1)
   <223> ACETYLATION
<220>
   <221> MOD_RES
   <222> (13) .. (13)
   <223> AMIDATION
<400> 1
   Ser Tyr Ser Met Glu His Phe Arg Trp Gly Lys Pro Val
   1               5                   10
<210> 2
   <211> 8
   <212> PRT
   <213> Artificial Sequence
<220>
   <223> Tyrosinase inhibitor peptide
<400> 2
   Ile Ser Leu Leu Asp Ala Gln Ser
   1 5
<210> 3
   <211> 8
   <212> PRT
   <213> Artificial Sequence
<220>
   <223> Tyrosinase inhibitor peptide
<220>
   <221> MOD..RES
   <222> (1)..(1)
   <223> ACETYLATION
<400> 3
   Ile Ser Leu Leu Asp Ala Gln Ser
   1 5
<210> 4
   <211> 8
   <212> PRT
   <213> Artificial Sequence
<220>
   <223> Tyrosinase inhibitor peptide
<220>
   <221> LIMPID
   <222> (1) .. (1)
   <223> PALMITATE
<400> 4
   Ile Ser Leu Leu Asp Ala Gln Ser
   1 5

## Description

### FIELD OF THE INVENTION

The present invention relates to the field of pharmacy and cosmetics, in particular, it relates to targeted microcapsules or nanocapsules comprising a skin whitening compound and to their pharmaceutical use for the prevention and/or treatment of skin dark spots.

### BACKGROUND

In dermatology, hyperpigmentation is the darkening of an area of skin or nails caused by an increased or excess production of melanin. Melanin is produced by melanocytes at the lower layer of the epidermis. The production of melanin is called melanogenesis. There are three basic types of melanin: eumelanin, pheomelanin, and neuromelanin.

Melanocortin receptor 1 (MC1 R) is known as a peripheral receptor that is mainly found in different skin cells (melanocytes, keratinocytes, sebocytes, and others). MC1 R is involved in pigmentation and inflammation responses, and on various melanoma cells, and found to control the relative amounts of eumelanin and pheomelanin in mammals. (Bednarek, M. et al., Biopolymers, 2008, 89(5), 401-408).

α-Melanocyte Stimulating Hormone (α-MSH):
Ac-Ser¹-Tyr²-Ser³-Met⁴-Glu⁵-His⁶-Phe⁷-Arg⁸-Trp⁹-Gly¹⁰-Lys¹¹-Pro¹²-Val¹³-NH₂ (SEQ ID NO: 1)
is an endogenous agonist for the MC1 R.

From previous studies with α-MSH and its synthetic analogue (NDP-α-MSH):
Ac-Ser¹-Tyr²-Ser³-Nle⁴-Glu⁵-His⁶-D-Phe⁷-Arg⁸-Trp⁹-Gly¹⁰-Lys¹¹-Pro¹²-Val¹³-NH₂
it has been considered that segment 6-9 (His⁶-D-Phe⁷-Arg⁸-Trp⁹) is essential for the ligand-receptor interaction.

Analogues of the previous mentioned peptides were synthesized with the following various amino acids in place of Phe⁷ and Trp⁹ in NDP-α-MSH: Ala, Val, Cha, Lys, Glu, Phe, Pip, Pro, Oic, Tic, Sar, Gly. In view of the results Bednarek et al conclude that Trp⁹ residue of NDP-αMSH is not essential for molecular recognition at human melanocortin receptor 1 (hMC1bR).

WO 2008/107533 discloses a combination of at least one MC1 R receptor inhibitor, a tyrosinase inhibitor derived from vitamin C and an inhibitor of the transfer of melanosomes to the keratinocytes, to improve the activity of the tyrosinase inhibitor in the treatment of dark spots on the skin. It also describes the use of the combination in cosmetics and dermatology for preparing whitening and/or bleaching depigmentation compositions.

Among the MC1-R receptor inhibitors WO 2008/107533 lists the Agouti protein, Melanostatine ®5 (INCI name: nonapeptide-1) and the lipo amino acid undecylenoyl phenylalanine commercialized under the name Sepiwhite MSH ®.

Melanostatine ®5 consist in a synthetic nonapeptide of the following amino acids: Arg-Lys-Met-D-Phe-Phe-Pro-Pro-Trp-Val.

Sepiwhite is the commercial name of N-undecyl-10-enoyl-L-phenylalanine. Sepiwhite is a reported alpha-melanocyte-stimulating hormone (α-MSH) receptor antagonist which reduces melanin production in cultured melanocytes.

Among the tyrosinase inhibitors derived from vitamin C WO 2008/107533 cites the esthers of ascorbic acid, for example, the esther of 2-glucoside ascorbic acid (INCI name: Ascorbyl Glucoside, AA-2G), 2-O-α-D-glucopyranosyl-6-O-hexadecanoyl-L-ascorbic acid, ascorbyl 6-palmitate, sodium or magnesium 2-phosphate ascorbic acid salt.

As inhibitors of the transfer of melanosomes, WO 2008/107533 describes the Nicotinamide (INCI name: niacinamide) and PP vitamin. It particularly describes a composition comprising niacianimide (0.01%-5%), ascorbyl glucoside (0.01%-5%) and melanostatine ® (0.1-40 ppm).

It has been reported that the combination of N-undecyl-10-enoyl-L-phenylalanine (Sepiwhite) and niacinamide, is significantly more effective than the niacinamide alone in reducing facial hyperpigmentation. Niacinamide has been found to inhibit melanosome transfer in cultured cells and to reduce the appearance of hyperpigmented spots in clinical studies. (Bissett, D.L.,J. Cosmet Dermatol, 2009; 8(4) 260-6).

However, the skin whitening products known in the art, namely the tyrosinase inhibitors, do not get in an efficient manner to the melanocytes.

### BRIEF DESCRIPTION OF THE INVENTION

The inventors have surprisingly found that a MC1 receptor agonist, i.e. the peptide of formula (I):

R₂-Ser¹-Tyr²-Ser³-Nle⁴-Glu⁵-His⁶-DPhe⁷-Arg⁸-(AA₁)⁹-Gly¹⁰-Lys¹¹-DPro¹²-Val¹³-R₁ (I)

which is a compound capable of selectively binding to MCR1 receptors on the melanocyte, in combination with a compound able to inhibit the activity or expression of tirosinase, i.e. a skin whitening compound, is useful for the improvement in the prevention and/or treatment of cutaneous dark spots as the tyrosinase inhibitors get to the melanocytes in an efficient manner.

One of the possible combinations of the peptide of formula (I) and the skin whitening compound is by forming a capsule, that may be a microcapsule or a nanocapsule, which encapsulates the skin whitening agent and wherein the peptide of formula (I) is coupled to the outer surface of the capsule which allows targeting the MCR1 receptors on the melanocyte, i.e. the inventors have developed targeted capsules for the delivery of skin whitening compounds, which improve the whitening effect of the skin whitening compounds. These are surprising results as the peptide of formula (I) is a MC1 receptor agonist, and not a MC1 R receptor inhibitor which are normally used in the art for the effect of improving the activity of the tyrosinase inhibitor, i.e. skin whitening compound, in the treatment of dark spots on the skin, e.g. Melanostatine ®5 and Sepiwhite MSH ®.

The microcapsules or a nanocapsules of the invention have the advantage that they reach deep into the hair follicles, where the barrier possess only a few layers of differentiated corneocytes and can be considered highly permeable. Additionally, the hair follicles can act as long-term reservoir, beneficial condition when transdermal delivery is intended. The hair follicle delivery has several pharmacokinetic advantages as a reduction or bypass of the tortuous pathway of the transepidermal absorption, decrease of the drug systemic toxicity when the follicle acts as long term delivery reservoir and increasing additionally the therapeutic index of some drugs as well as reducing the applied dose or frequency of administration.

The targeted microcapsules or nanocapsules, and more particularly the targeted bilayered microcapsules or nanocapsules, can be used as a highly efficient delivery system with sustained and controlled release of the encapsulated active ingredient at the target cells: fibroblasts and keratinocytes. In particular, the microcapsules or nanocapsules escape from the endosomal and lysosomal compartment so that the encapsulated active ingredient is released directly in the cytoplasm and reaches the target quickly, optimizing the application and the bioavailability of the encapsulated active agents.

In addition, the targeted microcapsules or nanocapsules of the invention have the advantage that they show higher migration and penetration into the skin when compared with conventional microcapsules.

Brief, the use of the targeted microcapsules or nanocapsules of the present invention allows decreasing the amount needed of the active ingredients with the consequence that potential side-effects are also reduced. Further, the microcapsules or nanocapsules have also the advantage that are capable of penetrating skin and show a uniformly distribution on the entire epidermis.

Therefore, one aspect of the present invention relates to a microcapsule or nanocapsule comprising a skin whitening compound and a peptide of formula (I)

R₂-Ser¹-Tyr²-Ser³-Nle⁴-Glu⁵-His⁶-DPhe⁷-Arg⁸-(AA₁)⁹-Gly¹⁰-Lys¹¹-DPro¹²-Val¹³-R₁ (I)

wherein
R₁, is selected from the group consisting of -OH, -OR₃, -SR₃, -NR₃R₄, -NHR₉, polyethylene glycol or a derivative thereof and -OR₉ wherein R₉ is polyethylene glycol or a derivative thereof;
R₂, is selected from the group consisting of H-, R₃-C(O)- and R₃-OC(O)-,
R₃ and R₄ are independently selected from H, C₁-C₂₄ alkyl, C₂-C₂₄ alkenyl and C₆-C₁₀ aryl;
AA₁, is selected independently from the amino acid groups that represents an aromatic amino acid group, preferably selected from the group consisting of tryptophan (including L-tryptophan and D-tryptophan), 3-(2-Naphthyl)-D-alanine, 3-(1-Naphthyl)-D-alanine, 3-amino-3-(1-naphthyl) propionic acid, 3-amino-3-(biphenyl)-propionic acid, phenylalanine, tyrosine, histidine, 5-hydroxytryptophan, D-1,2,3,4-tetrahydroisoquinoline-3-carboxylic acid and L-3,4-dihydroxyphenylalanine, more preferably selected from the group consisting of tryptophan (including L-tryptophan and D-tryptophan), 3-(2-Naphthyl)-D-alanine, 3-amino-3-(1-naphthyl) propionic acid, 3-amino-3-(biphenyl)-propionic acid, phenylalanine, tyrosine, histidine, 5-hydroxytryptophan and L-3,4-dihydroxyphenylalanine;
and cosmetically acceptable salts and solvates thereof, coupled to the outer surface of the microcapsule or nanocapusle.

This invention makes use at a compound of formula (I)

R₂-Ser¹-Tyr²-Ser³-Nle⁴-Glu⁵-His⁶-DPhe⁷-Arg⁸-(AA₁)⁹-Gly¹⁰-Lys¹¹-DPro¹²-Val¹³-R₁ (I)

wherein
R₁, is selected from the group consisting of -OH, -OR₃, -SR₃, -NR₃R₄, - NHR₉, polyethylene glycol or a derivative thereof and -OR₉ wherein R₉ is polyethylene glycol or a derivative thereof;
R₂, is selected from the group consisting of H-, R₃-C(O)- and R₃-OC(O)-,
R₃ and R₄ are independently selected from H, C₁-C₂₄ alkyl, C₂-C₂₄ alkenyl and C₆-C₁₀ aryl;
AA₁, is selected independently from the amino acid groups that represents an aromatic amino acid group, preferably selected from the group consisting of 3-(2-Naphthyl)-D-alanine, 3-(1-Naphthyl)-D-alanine, 3-amino-3-(1-naphthyl) propionic acid, 3-amino-3-(biphenyl)-propionic acid, phenylalanine, tyrosine, histidine, 5-hydroxytryptophan, D-1,2,3,4-tetrahydroisoquinoline-3-carboxylic acid and L-3,4-dihydroxyphenylalanine, more preferably selected from the group consisting of 3-(2-Naphthyl)-D-alanine, 3-amino-3-(1-naphthyl) propionic acid, 3-amino-3-(biphenyl)-propionic acid, phenylalanine, tyrosine, histidine, 5-hydroxytryptophan, and L-3,4-dihydroxyphenylalanine, i.e. AA₁ is not tryptophan; and
salts and solvates thereof, preferably cosmetically acceptable salts thereof.

Another aspect refers to the process for obtention of the microcapsules and/or nanocapsule of the invention comprising the steps of
a) forming the microcapsule or nanocapsule encapsulating the withening compound and
b) coupling a compound of formula (I):

   R₂-Ser¹-Tyr²-Ser³-Nle⁴-Glu⁵-His⁶-DPhe⁷-Arg⁸-(AA₁)⁹-Gly¹⁰-Lys¹¹-DPro¹²-Val¹³-R₁ (I)

   wherein
   R₁, is selected from the group consisting of -OH, -OR₃, -SR₃, -NR₃R₄, - NHR₉, polyethylene glycol or a derivative thereof and -OR₉ wherein R₉ is polyethylene glycol or a derivative thereof;
   R₂, is selected from the group consisting of H-, R₃-C(O)- and R₃-OC(O)-,
   R₃ and R₄ are independently selected from H, C₁-C₂₄ alkyl, C₂-C₂₄ alkenyl and C₆-C₁₀ aryl;
   AA₁, is selected from the amino acid groups that represents an aromatic amino acid group prefereably selected from the group consisting of tryptophan (including L-tryptophan and D-tryptophan); 3-(2-Naphthyl)-D-alanine; 3-(1-Naphthyl)-D-alanine, 3-amino-3-(1-naphthyl) propionic acid; 3-amino-3-(biphenyl)-propionic acid, phenylalanine, tyrosine, histidine, 5-hydroxytryptophan, D-1,2,3,4-tetrahydroisoquinoline-3-carboxylic acid and L-3,4-dihydroxyphenylalanine more preferably selected from the group consisting of tryptophan (including L-tryptophan and D-tryptophan), 3-(2-Naphthyl)-D-alanine, 3-amino-3-(1-naphthyl) propionic acid, 3-amino-3-(biphenyl)-propionic acid, phenylalanine, tyrosine, histidine, 5-hydroxytryptophan and L-3,4-dihydroxyphenylalanine; and cosmetically acceptable salts and solvates thereof, to the outer surface of the microcapsule or nanocapsule before or after forming the microcapsule.

Another aspect of this invention refers to cosmetic composition comprising a microcapsule and/or nanocapsule as defined in the present invention.

Another aspect of this invention refers to the use of the peptide of formula (I) for the obtention of microcapsules or nanocapusles for the cosmetic prevention and/or cosmetic treatment of cutaneous dark spots.

Another aspect of this invention refers to the microcapsules or nanocapusles as defined in the present invention for the cosmetic prevention and/or cosmetic treatment of cutaneous dark spots.

This invention makes use of a peptide of formula (II):

R₅-Ile-Ser-Leu-Leu-Asp-Ala-Gln-Ser-R₆ [R₅-(SEQ ID NO: 2)-R₆] (II)

wherein
R₅ is selected from the group consisting of H-, R₇-C(O)- and R₇-OC(O)-,
R₆ is selected from the group consisting of -OH, -OR₇, -SR₇, and -NR₇R₈,
R₇ and R₈ are independently selected from H-, C₁-C₂₄ alkyl, C₂-C₂₄ alkenyl and C₆-C₁₀ aryl,
and cosmetically acceptable salts and solvates thereof.

Another aspect refers to the use of the peptide of formula (II) as defined above for the cosmetic prevention and/or cosmetic treatment of cutaneous dark spots.

Another aspect of this invention refers to compositions comprising a peptide of formula (II) and one or more whitening agent such as arbutine, vitamin C, ascorbyl phosphate, niacinamide, dynachondrine, a tyrosinase inhibitor peptide, tretinoin, hydroquinone, kojic acid and esters thereof with fatty acids, azelaic acid, glutathione, cinnamomum subavenium extracts, alpha-hydroxy acids such as lactic acid or glycolic acid, niacinamide, ferulic acid, vitamin E, ellagic acid, pomegrate extracts and mixtures thereof.

The invention is defined by the appended claims.

### BRIEF DESCRIPTION OF THE FIGURES

- Figure 1. :: % of melanin content in melanogenic conditions
- Figure 2:: % cell viability in melanogenic conditions
- Figure 3.: Binding to melanocytes for 1 h at 4 °C
- Figure 4.: Uptake by melanocytes for 1 h at 37 °C
- Figure 5.: Capsule selectivity for melanocytes in a mixed cell population

### DETAILED DESCRIPTION OF THE INVENTION

In the context of the present invention, the following terms have the meaning detailed below.

The terms "microcapsule" and "nanocapsule", also called herein "capsules", refer to capsules with a size distribution from 10 to 10000 nm. In one embodiment the capsules have a size distribution from 50 to 5000 nm, from 100 to 1000 nm, preferably from 150 to 450 nm and more preferably from 180 to 400 nm. This size allows the capsule to be uptaken by the cells, and to release the actives into the cytosol. In a particular embodiment the average size of the capsules is 220 nm as determined by Scanning Electron Microscopy (SEM).

In the context of the present invention whenever an aminoacid is mentioned without specifying its stereochemistry it is to be understood that the mention designates both the D and the L stereosiomers. For example, any mention to Leucine (or Leu) designates both D-Leucine and L-Leucine. When a specific stereoisomery is intented, this is indicated, for example D-Ala is used to designated the D stereoisomer of Alanine.

In one preferred embodiment, the microcapsules of the invention are polymeric microcapsules, generally made of one or more biodegradable polymers.

In one embodiment, the polymers forming the microcapsules of the invention are selected from the group consisting of poly(D,L-lactide-co-glycolide), polylactic acids, poly(propylene fumarate-co-ethylene glycol) [P(PF-co-EG)] block copolymer, poly-anhydride poly(fumaric-co-sebacic) anhydride, poly (ethylene oxide)-poly(lactide/glycolide), poly(amino acid), polyvinyl alcohol, alginate, dextran, chitosan, hydroxyapatite, collagen, fibrin, hyaluronic acid, carbomers, poly(amino acid) and poly(ethylene glycol).

In one embodiment, the microcapsules of the invention are bilayered polymeric microcapsules which comprise a core polymer (also called in the present invention "inner layer polymer"), and an outer shell polymer (also called in the present invention "outer layer polymer" or "polymer in the outer surface of the capsule"). The core polymers and the shell polymers are selected from the group consisting of poly(D,L-lactide-co-glycolide), polylactic acids, poly(propylene fumarate-co-ethylene glycol) [P(PF-co-EG)] block copolymer, poly-anhydride poly(fumaric-co-sebacic) anhydride, poly (ethylene oxide)-poly(lactide/glycolide), poly(amino acid), polyvinyl alcohol, alginate, dextran, chitosan, hydroxyapatite, collagen, fibrin, hyaluronic acid, carbomers, poly(amino acid) and poly(ethylene glycol). In a preferred embodiment the core polymer and the outer shell polymer are different. In a preferred embodiment, the core polymer is poly (D,L lactide-co-glycolide) (PLGA) and the outer shell polymer is polyvinyl alcohol (PVA). In a preferred embodiment, at least some of the functional groups of the core polymer, preferably when the functional groups are carboxyl groups of the core polymer, are present in the outer surface of the capsule, together with the polymer in the outer surface of the capsule. In a more preferred embodiment when the core polymer is poly (D,L lactide-co-glycolide) (PLGA) and the outer shell polymer is polyvinyl alcohol (PVA), the carboxyl groups from the PLGA polymer are present on the outside surface of the capsule. In a more preferred embodiment, PLGA has a lactide/glycolide molar ratio from 40:60 to 60:40, more preferably 50:50.

In other embodiment the microcapsules may be formed by lipids, liposomes, micelles, nanomaterials, dendrimers and the like.

In one embodiment the peptide of formula (I) is attached to the outer shell polymer on the surface of the microcapsule or nanocapsule. In a more particular embodiment, the peptide of formula (I) is attached to the outer shell polymer by a covalent bond. In a preferred embodiment the covalent bond is an amide bond between the amino group of the peptide's N-terminal group and the carboxyl group from the PLGA polymer present outside of the surface. Preferably, the outer shell polymer is polyvinyl alcohol.

The term "alkyloyl" is the same as alkanoyl, and refers to a radical having the general formula RCO- wherein R is an alkyl group.

The term "alkenyloyl" is the same as alkenoyl, and refers to a radical having the general formula RCO- wherein R is a alkenyl group.

"Alkyl" groups may be branched or unbranched, and preferably have from 1 to about 24 carbon atoms. One more preferred class of alkyl groups has from 1 to about 16 carbon atoms and a more preferred class of alkyl groups has from 1 to 6 and 14 to 18. Even more preferred are alkyl groups having 1, 2, 3, 4, 15, 16 or 17 carbon atoms. Methyl, ethyl, n-propyl, isopropyl and butyl, including n-butyl, tert-butyl, sec-butyl, isobutyl, pentadecyl, hexadecyl, heptadecyl are particularly preferred alkyl groups in the compounds of the present invention. Another preferred class of alkyl groups has from 6 to about 10 carbon atoms; and even more preferably 7, 8 or 9 carbon atoms. Heptyl, octyl and nonyl are the most preferred alkyl groups of this class.

The term "C₂-C₁₂ alkenyl" means a linear or branched hydrocarbon chain radical having one or more carbon-carbon double bonds therein and having from two to twelve carbon atoms, and which is attached to the rest of the molecule by a single bond. The double bond of an alkenyl group can be unconjugated or conjugated to another unsaturated group. Suitable alkenyl groups include, but are not limited to alkenyl groups such as vinyl, allyl, butenyl (e.g. 1-butenyl, 2-butenyl, 3-butenyl), pentenyl (e.g. 1-pentenyl, 2-pentenyl, 3-pentenyl, 4-pentenyl,), hexenyl (e.g. 1-hexenyl, 2-hexenyl, 3-hexenyl, 4-hexenyl, 5-hexenyl,), butadienyl, pentadienyl (e.g. 1,3-pentadienyl, 2,4-pentadienyl), hexadienyl (e.g. 1,3-hexadienyl, 1,4-hexadienyl, 1,5-hexadienyl, 2,4-hexadienyl, 2,5-hexadienyl), 2-ethylhexenyl (e.g. 2-ethylhex-1-enyl, 2-ethylhex-2-enyl, 2-ethylhex-3-enyl, 2-ethylhex-4-enyl, 2-ethylhex-5-enyl,), 2-propyl-2-butenyl, 4,6-dimethyl-oct-6-enyl. An alkenyl group can be unsubstituted or substituted with one or two suitable substituents.

"Aryl" herein refers to single and double ring radicals from 6 to about 10 carbon ring atoms, such as phenyl, naphthyl or indenyl. The aryl radical may be optionally substituted by one or more substituents such as hydroxy, mercapto, halo, alkyl, phenyl, alkoxy, haloalkyl, nitro, cyano, dialkylamino, aminoalkyl, acyl, alkoxycarbonyl, etc.

A "derivative of polyethylene glycol" in this invention are selected from monofunctional, homobifuntional or heterobifunctional poly (ethylene glycol) (PEG), otherwise known as poly(oxyethylene) or poly(ethylene oxide) (PEO), derivatives in a range of molecular weights, containing a single functional group, or two functional groups of the same or of different type, of various functionalities, such as amines, maleimides, azides, NHS esters and thiols. The derivatives of PEG are preferably selected from the group consisting of O,O'-Bis(3-aminopropyl)diethylene glycol, poly(ethylene glycol) bis(3-aminopropyl), poly(ethylene glycol) bis(amine), methoxypolyethylene glycol amine, methoxypolyethylene glycol azide, poly(ethylene glycol) bisazide, O-(2-Azidoethyl)-O'-methyl-triethylene glycol, O-(2-Azidoethyl)-O'-methyl-undecaethylene glycol, poly(ethylene glycol) methyl ether azide, poly(ethylene glycol) methyl ether, polyethylene glycol monomethyl ether mesylate, poly(ethylene glycol) methyl ether methacrylate, O-(2-Mercaptoethyl)-O'-methylpolyethylene glycol, poly(ethylene glycol) methyl ether thiol, poly(ethylene glycol) methyl ether tosylate, poly(ethylene glycol) methyl ether 2-bromoisobutyrate, poly(ethylene glycol)methyl ether acetylene. In a preferred embodiment R₉ is O,O'-Bis(3-aminopropyl)diethylene glycol (also called 4,7,10-Trioxa-1,13-tridecanediamine).

In a particular embodiment R₁ is selected from 4,7,10 -Trioxa-1,13-tridecanediamine, indicated as NH-PEG-NH₂ in the compound of formula (I) as follows:

R₂-Ser¹-Tyr²-Ser³-Nle⁴-Glu⁵-His⁶-DPhe⁷-Arg⁸-(AA₁)⁹-Gly¹⁰-Lys¹¹-DPro¹²-Val¹³-NH-PEG-NH₂

In a particular embodiment R₂ is selected from acetyl, propanoyl, pentadecanoyl, hexadecanoyl and heptadecanoyl. In a preferred embodiment R₂ is selected from hexadecanoyl (also called palmitoyl).

In a particular embodiment R₃ and R₄ are independently selected from H, and C₁₅, C₁₆ and C₁₇alkyl.

AA₁, is an amino acid selected from aromatic amino acids. In a particular embodiment, the peptide of formula (I) is attached to the microcapsule of the invention, and AA₁ is selected from the group consisting of tryptophan (including L-tryptophan and D-tryptophan), 3-(2-Naphthyl)-D-alanine, 3-(1-Naphthyl)-D-alanine, 3-amino-3-(1-naphthyl) propionic acid, 3-amino-3-(biphenyl)-propionic acid, phenylalanine, tyrosine, histidine, 5-hydroxytryptophan, D-1,2,3,4-tetrahydroisoquinoline-3-carboxylic acid and L-3,4-dihydroxyphenylalanine

In a preferred embodiment AA₁ is selected from the group consisting of tryptophan, 3-(2-Naphthyl)-D-alanine, 3-amino-3-(1-naphthyl) propionic acid, 3-amino-3-(biphenyl)-propionic acid, phenylalanine, tyrosine, histidine, 5-hydroxytryptophan, and L-3,4-dihydroxyphenylalanine.

In a particularly preferred embodiment AA₁ is selected from the group consisting of tryptophan, 3-(2-Naphthyl)-D-alanine, 3-amino-3-(1-naphthyl) propionic acid and, 3-amino-3-(biphenyl)-propionic acid.

The cosmetically acceptable salts of the peptides provided by this invention are also within the scope of the present invention. The term "cosmetically acceptable salts" means a salt generally admitted for its use in animals and more particularly in human beings, and includes the salts used to form base addition salts, either inorganic base addition salts, such as for example and in a non-limiting sense, lithium, sodium, potassium, calcium, magnesium or aluminium among others, or organic base addition salts, such as for example and in a non-limiting sense ethylamine, diethylamine, ethylenediamine, ethanolamine, diethanolamine, arginine, lysine, histidine or piperazine among others, or acid addition salts, either organic acid addition salts, such as for example and in a non-limiting sense acetate, citrate, lactate, malonate, maleate, tartrate, fumarate, benzoate, aspartate, glutamate, succinate, oleate, trifluoroacetate, oxalate, pamoate or gluconate among others, or inorganic acid addition salts, such as for example and in a non-limiting sense chloride, sulfate, borate or carbonate among others. The nature of the salt is not critical, provided that it is cosmetically acceptable. The cosmetically acceptable salts of the peptide derivatives of the invention can be obtained by conventional methods well known in the state of the art [Berge S.M., Bighley L.D. and Monkhouse D.C. (1977) "Pharmaceutical Salts" J. Pharm. Sci. 66:1-19].

The term "skin whitening compound" or "skin whitening agent" refers to a compound able to light and/or delete dark spots on the skin. The present invention refers as whitening compounds to compounds that inhibit the activity or expression of tirosinase, i.e. in an embodiment of the present invention a "skin whitening compound" is a "tirosinase inhibitor". In one embodiment the skin whitening compound is selected from the group consisting of arbutin, vitamin C, sodium ascorbyl phosphate, niacinamide, preferably niacinamide PC, a tyrosinase inhibitor peptide and mixtures thereof. In a preferred embodiment the skin whitening compound is selected from the group consisting of arbutin, sodium ascorbyl phosphate, vitamin C, niacinamide PC, a tyrosinase inhibitor peptide, tretinoin, hydroquinone, kojic acid and esters thereof with fatty acids, azelaic acid, glutathione, cinnamomum subavenium extracts, alpha-hydroxy acids such as lactic acid or glycolic acid, niacinamide, ferulic acid, vitamin E, ellagic acid, pomegrate extracts and mixtures thereof.

In a particular embodiment the mixture of skin whitening compounds is selected from mixture of two skin whitening compounds, more preferably the mixture of skin whitening compounds is selected from a mixture of arbutin and sodium ascorbyl phosphate and a mixture of niacinamide and sodium ascorbyl phosphate.

In a preferred embodiment the skin whitening compound or each skin whitening compound of the mixture of skin whitening compounds is present in an amount of about 0.1-30% wt in respect of the total weight of the capsule, preferably in an amount of about 5-25% wt in respect of the total weight of the capsule, more preferably in an amount of about 10-20% wt in respect of the total weight of the capsule. In a particular embodiment, the skin whitening compound or the mixture of whitening compounds is present in an amount of 20% wt in respect of the total weight of the capsule. In a more particular embodiment, the mixture of two skin whitening compounds is present in an amount of about 20%wt, preferably 10%wt each skin whitening compound. In one embodiment, the microcapsule comprises from 10 to 30% in weight of arbutin or niacinamide in relation to the total weight of the microcapsule. In one embodiment, the microcapsule comprises from 0.1 % to 10%, preferably from 0.1 to 3% of the peptide of formula (II) in relation to the total weight of the microcapsule.

Another aspect of the present invention relates to a tyrosinase inhibitor peptide of formula (II). The peptide of formula (II) acts intracellularly blocking or inhibiting the activity of the tirosinase

Preferably the tyrosinase inhibitor peptide is a peptide of formula (II):

R₅-Ile-Ser-Leu-Leu-Asp-Ala-Gln-Ser-R₆ R₅-[(SEQ ID NO: 2)-R₆] (II)

wherein
R₅ is selected from the group consisting of H-, R₇-C(O)- and R₇-OC(O)-,
R₆ is selected from the group consisting of -OH, -OR₇, -SR₇, and -NR₇R₈,
R₇ and R₈ are independently selected from H-, C₁-C₂₄ alkyl, C₂-C₂₄ alkenyl and C₆-C₁₀ aryl,
and cosmetically acceptable salts and solvates thereof.

In one embodiment the tyrosinase inhibitor peptide is a peptide of formula:

R₅-Ile-Ser-D-Leu-Leu-Asp-D-Ala-Gln-Ser-R₆

wherein
R₅ is selected from the group consisting of H-, R₇-C(O)- and R₇-OC(O)-,
R₆ is selected from the group consisting of -OH, -OR₇, -SR₇, and -NR₇R₈,
R₇ and R₈ are independently selected from H-, C₁-C₂₄ alkyl, C₂-C₂₄ alkenyl and C₆-C₁₀ aryl,
and cosmetically acceptable salts and solvates thereof

In one embodiment R₇ and R₈ are H.

In one embodiment R₇ is C₁-C₂₄ alkanoyl, prefereably a C₁-C₂₄ alkanoyl selected from the group consisting of acetyl, propanoyl, butanoyl, pentanoyl, hexanoyl, heptanoyl, octanoyl, nonanoyl, decanoyl, undecanoyl, dodecanoyl, tridecanoyl, tetradecanoyl, pentadecanoyl, hexadecanoyl, heptadecanoyl, octadecanoyl, nonadecanoyl and icosanoyl. In a preferred embodiment the alkanoyl selected from the group consisting of acetyl, propanoyl, pentadecanoyl, hexadecanoyl and heptadecanoyl. In one embodiment R₅ is acetyl (Ac). In another embodiment R₅ is hexadecanoyl (or commonly named palmitoyl (Palm)).

In one embodiment, when R₆ is -NR₇R₈, R₇ and R₈ are H.

In one embodiment the peptide of formula (II) is selected from the group:

Ac-Ile-Ser-DLeu-Leu-Asp-DAla-Gln-Ser-NR₇R₈

Ac-Ile-Ser-Leu-Leu-Asp-DAla-Gln-Ser-NR₇R₈

Ac-Ile-Ser-Leu-Leu-Asp-Ala-Gln-Ser-NR₇R₈ [(SEQ ID NO: 3)-NR₇R₈]

Palm-Ile-Ser-Leu-Leu-Asp-DAla-Gln-Ser-NR₇R₈
Palm-Ile-Ser-Leu-Leu-Asp-Ala-Gln-Ser-NR₇R₈[(SEQ ID NO: 4)-NR₇R₈]
Palm-Ile-Ser-DLeu-Leu-Asp-DAla-Gln-Ser-NR₇R₈,
and cosmetically acceptable salts and solvates thereof,
wherein R₇ and R₈ are independently selected from H, methyl, ethyl, propyl and butyl.

A "dark spot on the skin" or "cutaneous dark spot" herein refers to hiperpigmentation of the skin caused by the increase of melanin. Hyperpigmentation can be diffuse or focal.

The "cosmetic treatment" of "dark spot on the skin" or "cutaneous dark spot" refers to lightening dark spots on the skin, eliminating (i.e. deleting) dark spots on the skin, reducing the size of dark spots on the skin and/or reducing the number of dark spots on the skin.

The "cosmetic prevention" of "dark spot on the skin" or "cutaneous dark spot" refers to prevent the appearance of dark spots on the skin, prevent the darkening of already existing dark spots on the skin, and /or prevent the increase of the size of already existing dark spots on the skin.

Another aspect of the present invention relates to compounds of general formula (I), wherein AA₁ is not Trp.

Another aspect of the present invention relates to a process for the preparation of microcapsules or nanocapsules of the invention, comprising the steps of:
a) forming the microcapsule or nanocapsule encapsulating the skin whitening compound and
b) coupling a compound of formula (I) of the invention, including when AA₁ is Trp, to the outer surface of the microcapsule or nanocapsule before or after forming the microcapsule or nanocapsule.

In a preferred embodiment the step of coupling a compound of formula (I) of the invention, including when AA₁ is Trp, to the outer surface of the microcapsule or nanocapsule is done once the microcapsule or nanocapsule is formed, i.e. after forming the microcapsule or nanocapsule.

In a preferred embodiment, wherein the microcapsule or nanocapsule is a polymeric bilayered microcapsule or nanocapsule, the step of forming the microcapsule or nanocapsule encapsulating the withening compound comprises the steps of
a1) mixing the inner layer polymer with the skin whitening compound in a suitable solvent,
a2) emulsifying the mixture obtained in step a) with the outer layer polymer in a suitable solvent, and optionally
a3) isolating the microcapsules.

In one embodiment wherein the microcapsule or nanocapsule is a polymeric bilayered microcapsule or nanocapsule the process of coupling the compound of formula (I) of the invention, including when AA₁ is Trp, to the outer layer polymer, when said polymer has carboxyl groups and the coupling in step b) comprises the steps of
b1) activating the carboxyl groups on the surface of the capsules, and
b2) reacting the activated capsules with the N-terminal amine group of the compound of formula (I).

In another embodiment the outer layer polymer has amino groups and the coupling in step b) comprises the steps of
b1) activating the amino groups on the surface of the capsules, and
b2) reacting the activated capsules with the C-terminal group of the compound of formula (I).

In another embodiment the outer layer polymer has maleimide groups and the coupling in step b) comprises the steps of
b1) activating the maleimide groups on the surface of the capsules,
b3) functionalizing the compound of formula (I) with a thiol group by adding an extra amino acid with such group or by modifying at least one of the functional groups of the present amino acids, and
b2) reacting the activated capsules with the thiol group of the compound of formula (I).

In one embodiment the suitable solvent for mixing the inner layer polymer with the skin whitening compound is selected from the group consisting of acetone, acetonitrile, dichloromethane (DCM), ethanol, methanol, chloroform, dimethylformamide (DMF), and ethylacetate, preferably acetone.

In one embodiment the suitable solvent for emulsifying the mixture obtained in step a1) with the outer layer polymer is selected from the group consisting of water, acetonitrile, dichloromethane (DCM), ethanol, methanol, chloroform, dimethylformamide (DMF), dimethylsulfide (DMS) and ethylacetate, preferably water, ethanol, methanol, dimethylformamide, and dimethylsulfide (DMS) and more preferably water.

Another aspect of the invention refers to a process for obtention of bilayered microcapsules or nanocapsules as defined in the invention comprising the steps of:
i) coupling a compound of formula (I) of the invention, including when AA₁ is Trp, to the polymer which serves as outer layer polymer,
ii) mixing the inner layer polymer with the skin whitening compound in a suitable solvent,
iii) emulsifying the polymer obtained in step i) with the mixture obtained in step ii) in a suitable solvent, and optionally
iv) isolating the microcapsules.

In a preferred embodiment the cosmetic composition comprising a microcapsule and/or nanocapsule of the invention comprises at least one cosmetically acceptable excipient or adjuvant.

The term excipients or adjuvants also relate to carriers. Such pharmaceutical excipients, adjuvants or carriers can be liquids, such as water, oils or surfactants, including those of petroleum, animal, plant or synthetic origin, such as for example and in a non-limiting sense peanut oil, soybean oil, mineral oil, sesame oil, castor oils, polysorbates, sorbitan esters, ether sulfates, sulfates, betaines, glucosides, maltosides, fatty alcohols, nonoxinols, poloxamers, polyoxyethylenes, polyethylene glycols, dextrose, glycerol and the like. "Remington's Pharmaceutical Sciences" by E.W. Martin describes diluents, adjuvants or excipients as suitable carriers.

The following examples are merely illustrative of certain embodiments of the invention and cannot be considered as restricting it in any way.

### EXAMPLES

### Example 1: Preparation of the microcapsules

1 g of poly (D,L lactide-co-glycolide) (PLGA) (Resomer RG 502 H, lactide/glycolide molar ratio 48:52 to 52:48) in 10 mL of acetone containing hydrolyzed soy protein (25 mL) (Dynachondrine™ ISR, Maymó batch number M.1261.10, ISP Pharmaceuticals batch number 0Y900000277) was added dropwise to 100 mL of aqueous 1% (w/v) polyvinyl alcohol (PVA), and the mixture was emulsified for 3 min using a sonicator. Following overnight evaporation of the solvent at 4 °C, the capsules were collected by ultracentrifugation at 60000 g for 30 min, washed three times with distilled water, and then lyophilized for 3 to 4 days.

The following peptides of sequence of formula (I):
Pamitoyl-Ser-Tyr-Ser-Nle-Glu-His-DPhe-Arg-Trp-Gly-Lys-DPro-Val-NH-PEG-NH₂
Acetyl-Ser-Tyr-Ser-Nle-Glu-His-DPhe-Arg-Trp-Gly-Lys-DPro-Val-NH-PEG-NH₂
wherein NH-PEG-NH₂ represents the group NH-(CH₂)₃-(OCH₂CH₂)₂-CH₂-NH₂.
were coupled by the N-terminus of the peptide (Ser) to the above obtained microcapsule via amide bound to the carboxyl groups on the surface of the capsules present on the surface of the microcapsule (belonging to the inner polymer). Thus, carboxyl groups on the surface of the capsules were activated by re-suspending the capsules in isotonic 0.1 M 2-(N-morpholino)ethanesulphonic acid (MES) saline buffer pH 5.5, and then reacting them with 1-ethyl-3-(3-dimethyl aminopropyl) carbodiimide (EDAC) (10 equiv.) and N-hydroxysuccinimide (NHS) (10 equiv.) for 1 h. The capsules were then centrifuged (15000 rpm, 45 min) to remove excess EDAC/NHS and the water-soluble isourea byproduct. Activated capsules (1 g) were re-suspended in phosphate-buffered saline (PBS, 40 mL) and reacted with the N-terminal amine group of the peptide of sequence of formula (I) (0.150 g) at room temperature for 2 hours. The coated capsules were centrifuged (15000 rpm, 30 min) and washed with PBS (3 x 10 mL) buffer to remove any unbound peptide. The presence of surface-bound peptide was confirmed by Kaiser ninhydrin tests. Unreacted peptide was separated from the capsules after the corresponding reaction step by analysis of the centrifugation supernatant (step described above) using 2,4,6-trinitrobenzenesulphonic acid (TNBS) as a colorimetric assay: 700 microliters of supernatant were added to 700 microliters of 0.1 M sodium borate buffer (pH 9.2). 350 microliter of TNBS aqueous solution (1.65 mg/mL) was added and the solution was rapidly mixed. After incubation at 40 °C for 45 min., the reaction was stopped by adding 350 microliter of 0.1 M NaH₂PO₄ containing 1.5 mM Na₂SO₃ and absorption at 420 nm was determined on a UV/VIS spectrometer. The amounts of peptide on the surface of the nanocapsules were calculated by substracting the free amount from the total amount added into the reaction system, being in that case 0.125%. The ratio of encapsulated hydrolyzed soy protein to peptide of sequence of formula (I) is 25 to 0.125.

The diameter of the capsules was determined by Scanning Electron Microscopy (SEM), showing a size distribution between 180 and 400 nm (average size 220 nm).

### Example 2. Cytotoxicity assay

Human dermal fibroblasts and human keratinocytes were used. The isolated cells were cultured in DMEM culture medium supplemented with 10% FCS and were incubated at 37 °C for maintenance treatment. Untreated cells were used as negative control. The positive control was 0.1% SDS. The microcapsules as obtained in Example 1 were tested in the DMEM culture medium at two concentrations: 0.03% and 0.3%. These weight percentages refer to the weight of the microcapsule in relation to the total weight.

Cells were seeded at 20000 cells per well in a 96-well plate. 24 hours after seeding, cells were incubated under the conditions described above. After 48h of incubation at 37 °C, the culture medium was removed and the MTT reagent was added. Cells were incubated 2 hours at 37 °C. DMSO was then added and the absorbance of the formed formazan salt was measured at λ 570 nm. Lower absorbance values correspond to cells with lower metabolic activity, which correlates with an increased damage and, therefore, with an increased cytotoxic effect. Thus, the amount of living cells is proportional to the amount of formazan produced. Table 1 shows the percentage of cell viability, as well as the standard deviation (SD), in the different cell lines after the different treatments.

**Table 1**

| **Tested product** | **Fibroblasts** | | **Keratinocytes** | |
|---|---|---|---|---|
| | **% cell viability** | **SD** | **% cell viability** | **SD** |
| Control | 100 | 3.90 | 100 | 2.98 |
| SDS | 30.0 | 3.00 | 13.0 | 2.00 |
| Example 1 (0.03%) | 99.8 | 4,00 | 100.0 | 3.00 |
| Example 1 (0.3%) | 102 | 2,00 | 104.2 | 3.10 |

As can be seen, the microcapsules of Example 1 showed no changes in the cell viability at the different tested concentrations. Thus, is can be concluded that the microcapsules of Example 1 are not cytotoxic in any of the cell lines employed.

### Example 3. Skin penetration assay

Fluorescein-labeled microcapsules were prepared following an analogous process as described in Example 1, by additionally adding the marker DQ-BSA to the acetone mixture containing PLGA and hydrolyzed soy protein.

Reconstituted skin (SkinEthic, RHE Reconstructed Human Epidermis) was cultured in DMEM culture medium supplemented with 10% FCS and incubated at 37 °C for maintenance and treatment. Untreated RHE was used as negative control. Formulations containing the labelled microcapsules with DQ-BSA were tested medium at three concentrations: 0.03%wt, 0.15%wt, 0.30%wt. These weight percentages refer to the weight of the microcapsule in relation to the total weight.

The reconstructed skin models of size 0.5 cm², 17 day, were reconstituted in the appropriate means provided by the supplier immediately upon arrival at laboratory and kept in the incubator at 37 °C for complete recovery. After 24 hours, treatment with the marked microcapsules was performed under the conditions detailed in the previous section. Incubation with the different treatments was carried out for 48 hours. Then, the samples were frozen and analyzed by fluorescence microscopy.

Images corresponding to a cross section of skin treated with a formulation containing the microcapsules as obtained in Example 1 at 0.15% were recorded. In an image taken by fluorescence microscopy, the green fluorescence signal indicated that the capsules penetrated and were uniformly distributed along the whole epidermis. The fluorescence did not appear in the nucleus of skin cells, but only in the cytoplasm where the capsule was degraded, DQ-BSA complex was cleaved and the fluorescent molecule released. In a comparative image obtained by light microscopy after hematoxylin-eosin staining, the nuclei of the skin cells were stained in a darker color.

In conclusion, the microcapsules of Example 1 showed a suitable penetration and a uniformly distribution on the entire epidermis.

### Example 4. Melanocyte culture and incubation procedure

Day 1 - Human melanocytes are rise by trypsinization and centrifuge at 1500 rpm (433 x g) in an Allegra X-22R (Beckman Coulter) centrifuge during 5 minutes, and then are resuspended in 1 ml of a complete culture medium to count the number of cells. A volume of 10 µl of the cells solution was dyed with Trypan Blue colorant and the cells are counted with a hemocytometer. Then a cell solution is prepared at a concentration of 2,5x10⁵ cells/ml in a complete medium and 100 µl of the cell suspension is seed in wells of 96 well plates. The plates are incubated at 37°C in a 5% CO₂ atmosphere.

Day 2 - The seed cells are washed and treated with the different products in basal conditions and promelanogenic conditions. For that, the medium is removed of wells and 100 ul of the products in basal medium with 0.5% of FBS is added. Previously, the test products are solubilised in the same medium. Arbutin is solubilised in ethanol.

The products are tested at 3 different concentrations in the presence and absence of the melanogenic inductor (100µM), i.e. isobutyl methyl-xantine (IBMX). The plates are incubated 37 °C and 5% CO₂ during 5 days. The experiments are carried out using 4 wells per condition. The melanin accumulation inside the cells is monitored daily by optical microscopy.

Days 3 and 4 - All treatments are renewed every 24 hours.

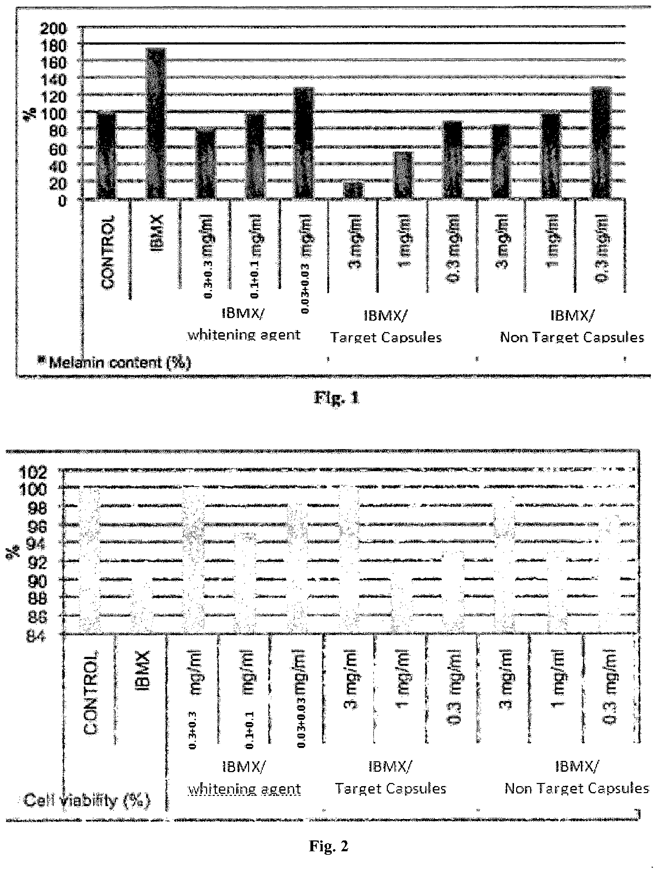

Day 5 - Determination of cell viability: In order to determine de number of viable cells, 10 µl of CCK -8 (WST-8) is added in each well. The plates are incubated at 37 °C during 1 hour and the absorbance is measured at 450 nm using Synergy II multimode (Biotek Instruments Inc., Winooski, Estados Unidos). The mode and SD is calculated for each concentration using Microsoft Excel application (see figure 2 and table 2)

### Example 5. Melanin determination

In order to determine the melanin content in cells treated as described in example 4, the medium is removed from each well and cells are washed with PBS 1 N and incubated during 3 days at 37°C facilitating the solubilisation of the melanin in NaOH.

Day 7 - Once the solubilisation of melanin is complete, 50µl de H₂O₂ MilliQ is added per well and the content is determined by absorbance using a Synergy II (Biotek Instruments Inc., Winooski, USA) at 390 nm. The mode and SD is calculated for each

**Table 2**

| **Product** | **Melanin content (%)** | **Cell viability** |
|---|---|---|
| Control | 100 | 100 |
| Isobutyl methyl-xantine (IBMX) | 174 ±12 | 90 |
| IBMX+ skin whitening agent (0.3 mg/ml of each) | 80 ± 8 | 100 |
| IBMX+ skin whitening agent (0.1 mg/ml of each) | 100 ± 9 | 95 |
| IBMX+ skin whitening agent (0.03 mg/ml of each) | 130 ± 12 | 98 |
| IBMX+ targeted capsules (3 mg/ml) | 18 ± 2 | 100 |
| IBMX+ targeted capsules (1 mg/ml) | 54 ± 6 | 91 |
| IBMX+ targeted capsules (0.3 mg/ml) | 90 ± 9 | 93 |
| IBMX+ non-targeted capsules (3 mg/ml) | 85 ± 9 | 99 |
| IBMX+ non targeted capsules (1 mg/ml) | 99 ± 10 | 93 |
| IBMX+ non-targeted capsules(0.3 mg/ml) concentration (see figure 1 and Table 2). | 130 ± 15 | 97 |

wherein:
IBMX means isobutyl methyl xantine. IBMX is used herein as is known that it induces melanogenesis, i.e. the synthesis of melanin.
"Skin whitening agent" in this example refers to a mixture 1:1, w/w of arbutin + sodium ascorbyl phospate. Concerning capsules, each component represents 10% of total weight meaning that 3 mg/ml Capsules contain 0.3 mg arbutin +0.3 mg sodium ascobyl phosphate.
"Targeted capsules" refers to the capsules of the invention as obtained in Example 1 comprising the skin whitening agent in a 20% wt in respect of the total weight of the capsule.
"Non-targeted capsules" refers to the capsules comprising the skin whitening agent without the peptide of formula (I).

The results show a decrease in the melanin content when the targeted capsules of the invention are used as compared to the non-encapsulated skin whitening compound and the encapsulated but non-targeted skin whitening compound.

### Example 6. Melanine determination and mushroom tyrosinase activity inhibition

The activity of several peptides of formula (II) (listed in table 3 below) to reduce melanine was determined as explained in example 5 after treating the cells as explained in example 4.

The activity of several peptides of formula (II) to inhibit tyrosinase activity was determined as explained below:

The assay was performed in 96-well plates; 100µl of reaction mixture containing PBS pH 6.5, 25 µl of L-Tyrosine (1,5mM) and 20µl of the compounds at different concentrations were dispensed in each well. Then, 15 U of Mushroom Tyrosinase (20 µl) was added to each well. A reaction mixture without addition of any compound was also assayed and used as maximum activity control. Wells without enzyme served as reagent blank (Negative Control). The assay was performed using compound concentration range from 0 to 100µg/ml.

Plates were incubated at 30°C for 10 min and the amount of L-DOPA produced in the reaction mixture was measured using the Synergy II plate reader (Biotek) at 490 nm.

The activity was expressed as % of the maximum considered 100%.

**Table 3**

| **SECUENCE** | **% INHIBITION TYROSINASE** | **% MELANINE CONTENT** |
|---|---|---|
| Ac-Ile-Ser-Leu-Leu-Asp-Ala-Gln-Ser-NH2 | 28 | 87 |
| Ac-Ile-Ser-Leu-Leu-Asp-(d)Ala-Gln-Ser-NH2 | 31 | 80 |
| Ac-Ile-Ser-(d)Lei-Leu-Asp-(d)Ala-Gln-Ser-NH2 | 40 | 68 |
| Palm-Ile-Ser-(d)Leu-Leu-Asp-(d)Ala-Gln-Ser-NH2 | 48 | 65 |
| Palm-Ile-Ser-Leu-Leu-Asp-(d)Ala-Gln-Ser-NH2 | 42 | 78 |
| Palm-Ile-Ser-Leu-Leu-Asp-Ala-Gln-Ser-NH2 | 39 | 85 |

### Example 7. Capsule binding and uptake to melanocytes

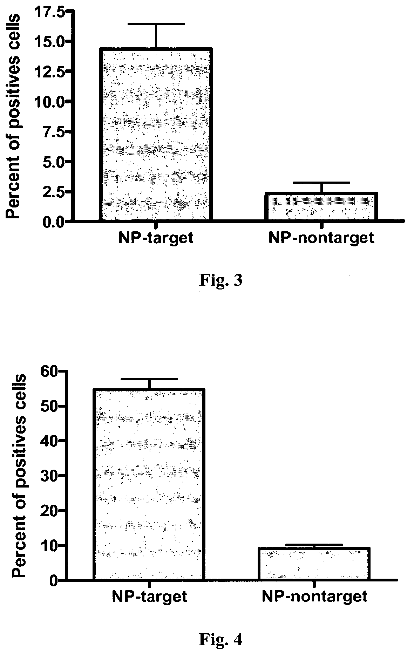

Analysis of binding and uptake of targeted capsules to melanocytes (Primary human epidermal melanocytes isolated from neonatal foreskin) using flow cytometry. Capsule binding was studied by incubating cells with 20 µg/mL, capsules targeted with Palm-Ser¹-Tyr²-Ser³-Nle⁴-Glu⁵-His⁶-DPhe⁷-Arg⁸-Trp⁹-Gly¹⁰-Lys¹¹-DPro¹²-Val¹³-NH-(CH₂)₃-(OCH₂CH₂)₂-CH₂-NH₂ or non-targeted capsules, for 1 h at 4°C in culture medium. Subsequently, cells were washed and analyzed by flow cytometry. Uptake was studied by incubating cells with 20 µg/mL target capsules or non-target capsules at 37°C for 1 h. The cells were washed and analyzed by flow cytometry on a FacsCalibur (Becton Dickinson, USA). See figures 3 and 4.

### Example 8. Targeting of capsules to melanocytes within a mixed cell population.

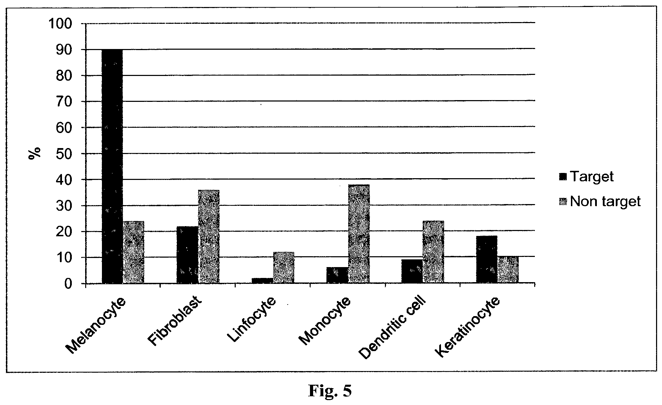

Distinct types of cells were incubated with the capsules of the invention (50 µg/mL) which have been targeted with different peptides of formula (Ia) or non-targeted capsules (50 µg/mL) at 37°C for 3 h. The peptides of formula (I) used to target the capsules in this example are peptides of formula Pamitoyl-Ser-Tyr-Ser-Nle-Glu-His-DPhe-Arg-(AA₁)-Gly-Lys-DPro-Val-NH-(CH₂)₃-(OCH₂CH₂)₂-CH₂-NH₂ wherein the nature of the aminoacid in position 9 (AA₁) is indicated in the first column of table 3. Cells were washed and stained with receptor-specific antibody or isotype control antibody to specifically detect distinct types of cells. Subsequently, cells were analyzed by flow cytometry on a FacsCalibur. The cell associated fluorescence was calculated by dividing the mean cell fluorescence intensity (mfi) for a given sample by the mfi of melanocytes incubated with targeted capsules, which was set at 100% (see figure 5 and table 4).

**Table 4**

| | **Cell specific binding (%)** | | | | | |
|---|---|---|---|---|---|---|
| **Peptide (AA₁)** | **Melanocyte** | **Fibroblast** | **Lymphocyte** | **Monocyte** | **Dendritic cell** | **Kerati-nocyte** |
| No peptide | 24 ± 2 | 18 ± 2 | 12 ± 1 | 8 ± 1 | 14 ± 2 | 10 ± 1 |
| Tryptophan | 90 ± 8 | 16 ± 2 | 2 ± 1 | 6 ± 1 | 9 ± 1 | 14 ± 2 |
| 3-(2-Naphthyl)-D-alanine | 71 ± 7 | 60 ± 5 | 10 ± 1 | 8 ± 1 | 10 ± 1 | 20 ± 2 |
| 3-amino-3-(1-naphthyl) propionic acid | 75 ± 7 | 23 ± 2 | 18 ± 2 | 7 ± 1 | 6 ± 1 | 16 ± 2 |
| 3-amino-3-(biphenyl) propionic acid | 74 ± 7 | 38 ± 4 | 42 ± 4 | 16 ± 2 | 27 ± 3 | 9 ± 2 |
| Phenylalanine | 85 ± 8 | 35 ± 4 | 45 ± 4 | 19 ± 2 | 7 ± 1 | 18 ± 2 |
| Tyrosine | 80 ± 8 | 30 ± 3 | 54 ± 5 | 20 ± 2 | 5 ± 1 | 34 ± 3 |
| Histidine | 58 ± 6 | 23 ± 2 | 18 ± 2 | 31 ± 3 | 16 ± 2 | 24 ± 3 |
| 5-hydroxytryptophan | 73 ± 7 | 21 ± 2 | 10 ± 1 | 14 ± 1 | 8 ± 1 | 15 ± 2 |
| L-3,4-dihydroxy-phenylalanine | 80 ± 8 | 15 ± 2 | 51 ± 5 | 26 ± 2 | 20 ± 2 | 85 ± 8 |

### Example 9. Peptide synthesis

In this Example AA refers to the amino acids of the peptide. The amino acids are those corresponding to the peptide of formula (I) or to the peptide of formula (II). Chemical peptide synthesis starts at the C-terminal end of the peptide and ends at the N-terminus. Therefore, the first AA is Val for the peptide of formula (I), and the first AA is Ser for peptide of formula (II).

Fmoc-AA-OH (3 eq) was directly incorporated on the-resin (0.5 mmol/g) with HBTU (3 eq), DIPEA (6 eq) in DMF for 1h. Washings were performed with DMF (5 x 30 s) and DCM (5 x 30 s). Kaiser test was used to verify that the coupling was successful. For the deprotection of the Fmoc group, the resin was solvated with DMF (5 x 30 s), treated with a solution of piperidine/DMF 20% (3 x 5 min) and finally washed with DMF (5 x 30 s) and DCM (5 x 30 s). Then, the resin was solvated with DMF (5 x 30 s). The same procedure was successively repeated n times with the following amino acids Fmoc-AA-OH. Finally, the cleavage of the peptide from the resin was carried out by treating the resin with TFA:TIS: H₂O (95:2.5:2.5) for 1 h, yielding the peptide of sequence HPLC: C18 column; UV 220 nm; flux 1mL/min; gradient acetonitrile-water in 8 min.

We describe:
[1]. A microcapsule or nanocapsule comprising a skin whitening compound and a peptide of formula (I):

   R₂-Ser¹-Tyr²-Ser³-Nle⁴-Glu⁵-His⁶-DPhe⁷-Arg⁸-(AA₁)⁹-Gly¹⁰-Lys¹¹-DPro¹²-Val¹³-R₁ (I)

   wherein
   R₁, is selected from the group consisting of -OH, -OR₃, -SR₃, -NR₃R₄, - NHR₉, polyethylene glycol or a derivative thereof and -OR₉ wherein R₉ is polyethylene glycol or a derivative thereof;
   R₂, is selected from the group consisting of H-, R₃-C(O)- and R₃-OC(O)-,
   R₃ and R₄ are independently selected from H, C₁-C₂₄ alkyl, C₂-C₂₄ alkenyl and C₆-C₁₀ aryl;
   AA₁, is selected from the amino acid groups that represents an aromatic amino acid group;
   and cosmetically acceptable salts and solvates thereof,
   coupled to the outer surface of the microcapsule or nanocapsule.
[2]. The microcapsule and/or nanocapsule according to [1] wherein the aromatic amino acid group is selected from the group consisting of tryptophan (including L-tryptophan and D-tryptophan), 3-(2-Naphthyl)-D-alanine, 3-(1-Naphthyl)-D-alanine, 3-amino-3-(1-naphthyl) propionic acid, 3-amino-3-(biphenyl)-propionic acid, phenylalanine, tyrosine, histidine, 5-hydroxytryptophan, D-1,2,3,4-tetrahydroiso-quinoline-3-carboxylic acid and L-3,4-dihydroxyphenylalanine.
[3]. The microcapsule and/or nanocapsule according to any [1] to [2] wherein the skin whitening compound is selected from the group consisting of arbutine, vitamin C, ascorbyl phosphate, niacinamide, dynachondrine, a tyrosinase inhibitor peptide, tretinoin, hydroquinone, kojic acid and esters thereof with fatty acids, azelaic acid, glutathione, cinnamomum subavenium extracts, alpha-hydroxy acids such as lactic acid or glycolic acid, ferulic acid, vitamin E, ellagic acid, pomegrate extracts and mixtures thereof.
[4]. The microcapsule and/or nanocapsule according to [3] wherein the skin whitening compound is a tyrosinase inhibitor peptide of formula (II):

   R₅-Ile-Ser-Leu-Leu-Asp-Ala-Gln-Ser-R₆ R₅-[(SEQ ID NO: 2)-R₆] (II)

   wherein
   R₅ is selected from the group consisting of H-, R₇-C(O)- and R₇-OC(O)-;
   R₆ is selected from the group consisting of -OH, -OR₇, -SR₇, and -NR₇R₈;
   R₇ and R₈ are independently selected from H-, C₁-C₂₄ alkyl, C₂-C₂₄ alkenyl and C₆-C₁₀ aryl;
   and cosmetically acceptable salts and solvates thereof.
[5]. The microcapsule and/or nanocapsule according to any of [1] to [4], characterized in that is a polymeric microcapsule or nanocapsule, preferably a bilayered polymeric microcapsule or nanocapsule, wherein the polymers forming the polymeric microcapsule and/or nanocapsule are selected from the group consisting of poly (D,L-lactide-co-glycolide), poly lactic acids, poly (propylene fumarate-co-ethylene glycol) [P(PF-co-EG)] block copolymer, poly-anhydride poly (fumaric-co-sebacic) anhydride, poly (ethylene oxide)-poly (lactide/glycolide), poly(amino acid), polyvinyl alcohol, alginate, dextran, chitosan, hydroxyapatite, collagen, fibrin, hyaluronic acid, carbomers, poly(amino acid), poly(ethylene glycol).
[6]. The microcapsule or nanocapsule according to [5], characterized in that the bilayered polymeric microcapsule and/or nanocapsule comprises poly (D,L-lactide-co-glycolide) as inner layer polymer and polyvinyl alcohol as outer layer polymer.
[7]. The microcapsule or nanocapsule according to any of [1] to [6], characterized in that the compound of formula (I) is covalently coupled to the outer layer polymer of the microcapsule.
[8]. The microcapsule or nanocapsule according to any of [1] to [7], characterized in that the size distribution of the microcapsule and/or nanocapsule is from 10 to 10000 nm.
[9]. A compound of formula (I)

   R₂-Ser¹-Tyr²-Ser³-Nle⁴-Glu⁵-His⁶-DPhe⁷-Arg⁸-(AA₁)⁹-Gly¹⁰-Lys¹¹-DPro¹²-Val¹³-R₁ (I)

   wherein
   R₁, is selected from the group consisting of -OH, -OR₃, -SR₃, -NR₃R₄, - NHR₉, polyethylene glycol or a derivative thereof and -OR₉ wherein R₉ is polyethylene glycol or a derivative thereof;
   R₂, is selected from the group consisting of H-, R₃-C(O)- and R₃-OC(O)-;
   R₃ and R₄ are independently selected from H, C₁-C₂₄ alkyl, C₂-C₂₄ alkenyl and C₆-C₁₀ aryl;
   AA₁, is selected independently from the amino acid groups that represents an aromatic amino acid group;
   and cosmetically acceptable salts and solvates thereof.
[10]. The compound according to [9] wherein the aromatic amino acid group is selected from the group consisting of 3-(2-Naphthyl)-D-alanine, 3-(1-Naphthyl)-D-alanine, 3-amino-3-(1-naphthyl) propionic acid, 3-amino-3-(biphenyl)-propionic acid, phenylalanine, tyrosine, histidine, 5-hydroxytryptophan, D-1,2,3,4-tetrahydroisoquinoline-3-carboxylic acid and L-3,4-dihydroxyphenylalanine.
[11]. A process for obtention of the microcapsules and/or nanocapsules as defined in [1] to [8] comprising the steps of
   a) forming the microcapsule or nanocapsule encapsulating the skin whitening compound and
   b) coupling a compound of formula (I):

      R₂-Ser¹-Tyr²-Ser³-Nle⁴-Glu⁵-His⁶-DPhe⁷-Arg⁸-(AA₁)⁹-Gly¹⁰-Lys¹¹-DPro¹²-Val¹³-R₁ (I)

      wherein
      R₁, is selected from the group consisting of -OH, -OR₃, -SR₃, -NR₃R₄, -NHR₉, polyethylene glycol or a derivative thereof and -OR₉ wherein R₉ is polyethylene glycol or a derivative thereof;
      R₂, is selected from the group consisting of H-, R₃-C(O)- and R₃-OC(O)-,
      R₃ and R₄ are independently selected from H, C₁-C₂₄ alkyl, C₂-C₂₄ alkenyl and C₆-C₁₀ aryl;
      AA₁, is selected from the amino acid groups that represents an aromatic amino acid group such as: tryptophan, 3-(2-Naphthyl)-D-alanine, 3-amino-3-(1-naphthyl) propionic acid, 3-amino-3-(biphenyl)-propionic acid, phenylalanine, tyrosine, histidine, thyrosine, 5-hydroxytryptophan, and L-3,4-dihydroxyphenylalanine;
   and cosmetically acceptable salts and solvates thereof,
   to the outer surface polymer of the microcapsule or nanocapsule before or after forming the microcapsule.
[12]. The process according to [11], wherein the microcapsule or nanocapsule is a polymeric bilayered microcapsule or nanocapsule having an inner layer polymer and an outer layer polymer and the step a) of forming the microcapsule or nanocapsule comprises the steps of
   a1) mixing the inner layer polymer with the skin whitening compound in a suitable solvent,
   a2) emulsifying the mixture obtained in step a) with the outer layer polymer in a suitable solvent, and optionally
   a3) isolating the microcapsules.
[13]. A cosmetic composition comprising the microcapsule and/or nanocapsule as defined in any of [1] to [8].
[14]. Use of the peptide of formula (I) as defined in [1] for the obtention of microcapsules or nanocapsules for the cosmetic prevention and/or cosmetic treatment of cutaneous dark spots.
[15]. Use of a microcapsule or nanocapsule as defined in any of [1] to [8] for the cosmetic prevention and/or cosmetic treatment of cutaneous dark spots.
[16]. A peptide of formula (II):

   R₅-Ile-Ser-Leu-Leu-Asp-Ala-Gln-Ser-R₆ R₅-[(SEQ ID NO: 2)-R₆] (II)

   wherein
   R₅ is selected from the group consisting of H-, R₇-C(O)- and R₇-OC(O)-,
   R₆ is selected from the group consisting of -OH, -OR₇, -SR₇, and -NR₇R₈,
   R₇ and R₈ are independently selected from H-, C₁-C₂₄ alkyl, C₂-C₂₄ alkenyl and C₆-C₁₀ aryl,
   and cosmetically acceptable salts and solvates thereof.
[17]. Use of the peptide of formula (II) as defined in [16] for the cosmetic prevention and/or cosmetic treatment of cutaneous dark spots.
[18]. A composition comprising a peptide of formula (II) as defined in [16] and one or more whitening agent selected from the group consisting of arbutine, vitamin C, ascorbyl phosphate, niacinamide, dynachondrine, a tyrosinase inhibitor peptide, tretinoin, hydroquinone, kojic acid and esters thereof with fatty acids, azelaic acid, glutathione, cinnamomum subavenium extracts, alpha-hydroxy acids such as lactic acid or glycolic acid, niacinamide, ferulic acid, vitamin E, ellagic acid, pomegrate extracts and mixtures thereof.

## Claims

1. A microcapsule or nanocapsule comprising a skin whitening compound selected from the group consisting of arbutine, vitamin C, ascorbyl phosphate, niacinamide, dynachondrine, a tyrosinase inhibitor peptide, tretinoin, hydroquinone, kojic acid and esters thereof with fatty acids, azelaic acid, glutathione, cinnamomum subavenium extracts, alpha-hydroxy acids such as lactic acid or glycolic acid, ferulic acid, vitamin E, ellagic acid, pomegrate extracts and mixtures thereof and a peptide of formula (I):
R₂-Ser¹-Tyr²-Ser³-Nle⁴-Glu⁵-His⁶-DPhe⁷-Arg⁸-(AA₁)⁹-Gly¹⁰-Lys¹¹-DPro¹²-Val¹³-R₁ (I)
wherein
R₁, is selected from the group consisting of -OH, -OR₃, -SR₃, -NR₃R₄, - NHR₉, polyethylene glycol or a derivative thereof and -OR₉ wherein R₉ is polyethylene glycol or a derivative thereof;
R₂, is selected from the group consisting of H-, R₃-C(O)- and R₃-OC(O)-,
R₃ and R₄ are independently selected from H, C₁-C₂₄ alkyl, C₂-C₂₄ alkenyl and C₆-C₁₀ aryl;
AA₁, is selected from the amino acid groups that represents an aromatic amino acid group;
and cosmetically acceptable salts and solvates thereof,
coupled to the outer surface of the microcapsule or nanocapsule.

2. The microcapsule and/or nanocapsule according to claim 1 wherein the aromatic amino acid group is selected from the group consisting of tryptophan (including L-tryptophan and D-tryptophan), 3-(2-Naphthyl)-D-alanine, 3-(1-Naphthyl)-D-alanine, 3-amino-3-(1-naphthyl) propionic acid, 3-amino-3-(biphenyl)-propionic acid, phenylalanine, tyrosine, histidine, 5-hydroxytryptophan, D-1,2,3,4-tetrahydroisoquinoline-3-carboxylic acid and L-3,4-dihydroxyphenylalanine.

3. The microcapsule and/or nanocapsule according to any one of claims 1 or 2 wherein the skin whitening compound is a tyrosinase inhibitor peptide of formula (II):
R₅-Ile-Ser-Leu-Leu-Asp-Ala-Gln-Ser-R₆ R₅-[(SEQ ID NO: 2)-R₆] (II)
wherein
R₅ is selected from the group consisting of H-, R₇-C(O)- and R₇-OC(O)-;
R₆ is selected from the group consisting of -OH, -OR₇, -SR₇, and -NR₇R₈;
R₇ and R₈ are independently selected from H-, C₁-C₂₄ alkyl, C₂-C₂₄ alkenyl and C₆-C₁₀ aryl;
and cosmetically acceptable salts and solvates thereof.

4. The microcapsule and/or nanocapsule according to any one of claims 1 to 3, **characterized in that** is a polymeric microcapsule or nanocapsule, preferably a bilayered polymeric microcapsule or nanocapsule, wherein the polymers forming the polymeric microcapsule and/or nanocapsule are selected from the group consisting of poly (D,L-lactide-co-glycolide), poly lactic acids, poly (propylene fumarate-co-ethylene glycol) [P(PF-co-EG)] block copolymer, poly-anhydride poly (fumaric-co-sebacic) anhydride, poly (ethylene oxide)-poly (lactide/glycolide), poly(amino acid), polyvinyl alcohol, alginate, dextran, chitosan, hydroxyapatite, collagen, fibrin, hyaluronic acid, carbomers, poly(amino acid), poly(ethylene glycol).

5. The microcapsule or nanocapsule according to claim 4, **characterized in that** the bilayered polymeric microcapsule and/or nanocapsule comprises poly (D,L-lactide-co-glycolide) as inner layer polymer and polyvinyl alcohol as outer layer polymer.

6. The microcapsule or nanocapsule according to any of claims 1 to 5, **characterized in that** the compound of formula (I) is covalently coupled to the outer layer polymer of the microcapsule.

7. The microcapsule or nanocapsule according to any of claims 1 to 6, **characterized in that** the size distribution of the microcapsule and/or nanocapsule is from 10 to 10000 nm.

8. A process for obtention of the microcapsules and/or nanocapsules as defined in claims 1 to 7 comprising the steps of
a) forming the microcapsule or nanocapsule encapsulating the skin whitening compound and
b) coupling a compound of formula (I):
R₂-Ser¹-Tyr²-Ser³-Nle⁴-Glu⁵-His⁶-DPhe⁷-Arg⁸-(AA₁)⁹-Gly¹⁰-Lys¹¹-DPro¹²-Val¹³-R₁ (I)
wherein
R₁, is selected from the group consisting of -OH, -OR₃, -SR₃, -NR₃R₄, -NHR₉, polyethylene glycol or a derivative thereof and -OR₉ wherein R₉ is polyethylene glycol or a derivative thereof;
R₂, is selected from the group consisting of H-, R₃-C(O)- and R₃-OC(O)-,
R₃ and R₄ are independently selected from H, C₁-C₂₄ alkyl, C₂-C₂₄ alkenyl and C₆-C₁₀ aryl;
AA₁, is selected from the amino acid groups that represents an aromatic amino acid group such as: tryptophan, 3-(2-Naphthyl)-D-alanine, 3-amino-3-(1-naphthyl) propionic acid, 3-amino-3-(biphenyl)-propionic acid, phenylalanine, tyrosine, histidine, thyrosine, 5-hydroxytryptophan, and L-3,4-dihydroxyphenylalanine;
and cosmetically acceptable salts and solvates thereof,
to the outer surface polymer of the microcapsule or nanocapsule before or after forming the microcapsule.

9. The process according to claim 8, wherein the microcapsule or nanocapsule is a polymeric bilayered microcapsule or nanocapsule having an inner layer polymer and an outer layer polymer and the step a) of forming the microcapsule or nanocapsule comprises the steps of
a1) mixing the inner layer polymer with the skin whitening compound in a suitable solvent,
a2) emulsifying the mixture obtained in step a) with the outer layer polymer in a suitable solvent, and optionally
a3) isolating the microcapsules.

10. A cosmetic composition comprising the microcapsule and/or nanocapsule as defined in any one of claims 1 to 7.

11. Use of the peptide of formula (I) as defined in claim 1 for the obtention of microcapsules or nanocapsules for the cosmetic prevention and/or cosmetic treatment of cutaneous dark spots.

12. Use of a microcapsule or nanocapsule as defined in any of claims 1 to 7 for the cosmetic prevention and/or cosmetic treatment of cutaneous dark spots.

## Patentansprüche

1. Eine Mikrokapsel oder Nanokapsel umfassend eine aus der aus Arbutin, Vitamin C, Ascorbylphosphat, Niacinamid, Dynachondrine, einem Tyrosinase-Inhibitor-Peptid, Tretionin, Hydrochinon, Kojisäure und ihren Estern mit Fettsäuren, Azelainsäure, Glutathion, Extrakte aus Cinnamomum subavenium, Alphahydroxysäuren wie etwa Milchsäure oder Glycolsäure, Ferulasäure, Vitamin E, Ellagsäure, Granatapfelextrakte und Mischungen daraus bestehenden Gruppe ausgewählte Hautaufhellungsverbindung und ein an die äußere Oberfläche der Mikrokapsel oder Nanokapsel gebundenes Peptid der Formel (I):
R₂-Ser¹-Tyr²-Ser³-Nle⁴-Glu⁵-His⁶-DPhe⁷-Arg⁸-(AA₁)⁹-Gly¹⁰-Lys¹¹-DPro¹²-Val¹³-R₁ (I)
wobei
R₁ aus der aus -OH, -OR₃, -SR₃, -NR₃R₄, -NHR₉, Polyethylenglycol oder einem Derivat davon und -OR₉ bestehenden Gruppe ausgewählt ist, wobei R₉ Polyethylenglycol oder ein Derivat davon ist;
R₂ aus der aus H-, R₃-C(O)- und R₃-OC(O)- bestehenden Gruppe ausgewählt ist;
R₃ und R₄ unabhängig voneinander aus H, C₁- bis C₂₄-Alkyl, C₂- bis C₂₄-Alkenyl und C₆-bis C₁₀-Aryl ausgewählt sind;
AA₁ aus den Aminosäuregruppen, die aromatische Aminosäuregruppen darstellen, ausgewählt ist;
sowie kosmetisch akzeptierbare Salze und Solvate davon.

2. Die Mikrokapsel und/oder Nanokapsel gemäß Anspruch 1, wobei die aromatische Aminosäuregruppe aus der aus Tryptophan (inklusive L-Tryptophan und D-Tryptophan), 3-(2-Naphthyl)-D-alanin, 3-(1-Naphthyl)-D-alanin, 3-Amino-3-(1-naphthyl)propionsäure, 3-Amino-3-(biphenyl)propionsäure, Phenylalanin, Tyrosin, Histidin, 5-Hydroxytryptophan, D-1,2,3,4-Tetrahydroisochonolin-3-carbonsäure und L-3,4-Dihydroxyphenylalanin bestehenden Gruppe ausgewählt ist.

3. Die Mikrokapsel und/oder Nanokapsel gemäß einem der Ansprüche 1 oder 2, wobei die Hautaufhellungsverbindung ein Tyrosinase-Inhibitor-Peptid der Formel (II) ist:
R₅-Ile-Ser-Leu-Leu-Asp-Ala-Gln-Ser-R₆ R₅-[(SEQ ID NO:2)-R₆] (II)
wobei
R₅ aus der aus H-, R₇-C(O)- und R₇-OC(O)- bestehenden Gruppe ausgewählt ist;
R₆ aus der aus -OH, -OR₇, -SR₇, und -NR₇R₈ bestehenden Gruppe ausgewählt ist;
R₇ und R₈ unabhängig voneinander aus H-, C₁- bis C₂₄-Alkyl, C₂- bis C₂₄-Alkenyl und C₆- bis C₁₀-Aryl ausgewählt sind;
sowie kosmetisch akzeptierbare Salze und Solvate davon.

4. Die Mikrokapsel und/oder Nanokapsel gemäß einem der Ansprüche 1 bis 3, **dadurch gekennzeichnet, dass** sie eine polymere Mikrokapsel oder Nanokapsel ist, bevorzugt eine doppelschichtige polymere Mikrokapsel oder Nanokapsel, wobei die die polymere Mikrokapsel und/oder Nanokapsel bildenden Polymere aus der aus Poly(D,L-lactid-co-glycolid), Polymilchsäuren, Poly(propylenfumarat-co-ethylenglycol)-Blockcopolymer [P(PF-co-EG)], Polyanhydrid Poly(fumarsäure-co-sebacinsäureanhydrid), Poly(ethylenoxid)-Poly(lactid/glycolide), Poly(aminosäure), Polyvinylaklohol, Alginat, Dextran, Chitosan, Hydroxyapatit, Collagen, Fibrin, Hyaluronsäure, Carbomere, Poly(aminosäure), Poly(ethylenglycol) bestehenden Gruppe ausgewählt sind.

5. Die Mikrokapsel oder Nanokapsel gemäß Anspruch 4, **dadurch gekennzeichnet, dass** die doppelschichtige polymere Mikrokapsel und/oder Nanokapsel Poly(D,L-lactid-co-glycolid) als Innenschichtpolymer und Polyvinylalkohol als Außenschichtpolymer umfasst.

6. Die Mikrokapsel oder Nanokapsel gemäß einem der Ansprüche 1 bis 5, **dadurch gekennzeichnet, dass** die Verbindung der Formel (I) kovalent an das Außenschichtpolymer der Mikrokapsel gebunden ist.

7. Die Mikrokapsel oder Nanokapsel gemäß einem der Ansprüche 1 bis 6, **dadurch gekennzeichnet, dass** die Größenverteilung der Mikrokapsel und/oder Nanokapsel von 10 bis 10.000 nm beträgt.

8. Ein Verfahren, um die Mikrokapseln und/oder Nanokapseln wie in Ansprüchen 1 bis 7 definiert zu erhalten, umfassend die Schritte
a) Bilden der Mikrokapsel oder Nanokapsel durch Einkapseln der Hautaufhellungsverbindung und
b) Binden einer Verbindung der Formel (I):
R₂-Ser¹-Tyr²-Ser³-Nle⁴-Glu⁵-His⁶-DPhe⁷-Arg⁸-(AA₁)⁹-Gly¹⁰-Lys¹¹-DPro¹²-Val¹³-R₁ (I)
wobei
R₁ aus der aus -OH, -OR₃, -SR₃, -NR₃R₄, -NHR₉, Polyethylenglycol oder einem Derivat davon und -OR₉ bestehenden Gruppe ausgewählt ist, wobei R₉ Polyethylenglycol oder ein Derivat davon ist;
R₂ aus der aus H-, R₃-C(O)- und R₃-OC(O)- bestehenden Gruppe ausgewählt ist;
R₃ und R₄ unabhängig voneinander aus H, C₁- bis C₂₄-Alkyl, C₂- bis C₂₄-Alkenyl und C₆-bis C₁₀-Aryl ausgewählt sind;
AA₁ aus den Aminosäuregruppen, die aromatische Aminosäuregruppen darstellen, wie etwa Tryptophan, 3-(2-Naphthyl)-D-alanin, 3-Amino-3-(1-naphthyl)propionsäure, 3-Amino-3-(biphenyl)propionsäure, Phenylalanin, Tyrosin, Histidin, Tyrosin, 5-Hydroxytryptophan und L-3,4-Dihydroxyphenylalanin, ausgewählt ist;
sowie kosmetisch akzeptierbare Salze und Solvate davon
an das Außenflächenpolymer der Mikrokapsel oder Nanokapsel, vor oder nach dem Bilden der Mikrokapsel.

9. Das Verfahren gemäß Anspruch 8, wobei die Mikrokapsel oder Nanokapsel eine polymere zweischichtige Mikrokapsel oder Nanokapsel mit einem Innenschichtpolymer und einem Außenschichtpolymer ist und der Schritt a) des Bildens der Mikrokapsel oder Nanokapsel folgende Schritte umfasst:
a1) Mischen des Innenschichtpolymers mit der Hautaufhellungsverbindung in einem geeigneten Lösungsmittel,
a2) Emulgieren der in Schritt a) erhaltenen Mischung mit dem Außenschichtpolymer in einem geeigneten Lösungsmittel und optional
a3) Isolieren der Mikrokapsel.

10. Eine kosmetische Zusammensetzung umfassend die Mikrokapsel und/oder Nanokapsel wie in einem der Ansprüche 1 bis 7 definiert.

11. Verwendung des Peptids der Formel (I) wie in Anspruch 1 definiert, um Mikrokapseln oder Nanokapseln für die kosmetische Vorbeugung gegen und/oder die kosmetische Behandlung von dunklen Hautflecken zu erhalten.

12. Verwendung einer Mikrokapsel oder Nanokapsel wie in einem der Ansprüche 1 bis 7 definiert für die kosmetische Vorbeugung gegen und/oder die kosmetische Behandlung von dunklen Hautflecken.

## Revendications

1. Microcapsule ou nanocapsule comprenant un composé blanchissant la peau choisi dans le groupe consistant en l'arbutine, la vitamine C, le phosphate d'ascorbyle, le niacinamide, la dynachondrine, un peptide inhibiteur de tyrosinase, la trétinoïne, l'hydroquinone, l'acide kojique et ses esters avec des acides gras, l'acide azélaïque, le glutathion, les extraits de cinnamomum subavenium, les alpha-hydroxyacides comme l'acide lactique ou l'acide glycolique, l'acide férulique, la vitamine E, l'acide ellagique, les extraits de grenade et les mélanges de ceux-ci et un peptide de formule (I) :
R₂-Ser¹-Tyr²-Ser³-Nle⁴-Glu⁵-His⁶-DPhe⁷-Arg⁸-(AA₁)⁹-Gly¹⁰-Lys¹¹-DPro¹²-Val¹³-R₁ (I)
où
R₁ est choisi dans le groupe consistant en -OH, -OR₃, -SR₃, -NR₃R₄,-NHR₉, le polyéthylèneglycol ou un dérivé de celui-ci et -OR₉ où R₉ est le polyéthylèneglycol ou un dérivé de celui-ci ;
R₂ est choisi dans le groupe consistant en H-, R₃-C(O)- et R₃-OC(O)-,
R₃ et R₄ sont choisis indépendamment parmi H, C₁-C₂₄ alkyle, C₂-C₂₄ alcényle et
C₆-C₁₀ aryle ;
AA₁ est choisi dans les groupes d'acides aminés qui représentent un groupe d'acides aminés aromatiques ;
et les sels acceptables du point de vue cosmétique et solvates de ceux-ci, couplé à la surface externe de la microcapsule ou nanocapsule.

2. Microcapsule et/ou nanocapsule selon la revendication 1 où le groupe d'acides aminés aromatiques est choisi dans le groupe consistant en le tryptophane (incluant le L-tryptophane et le D-tryptophane), la 3-(2-naphtyl)-D-alanine, la 3-(1-naphtyl)-D-alanine, l'acide 3-amino-3-(1-naphtyl)propionique, l'acide 3-amino-3-(biphényl)-propionique, la phénylalanine, la tyrosine, l'histidine, le 5-hydroxy-tryptophane, l'acide D-1,2,3,4-tétrahydroiso-quinoléine-3-carboxylique et la L-3,4-dihydroxyphénylalanine.

3. Microcapsule et/ou nanocapsule selon l'une quelconque des revendications 1 ou 2 où le composé blanchissant la peau est un peptide inhibiteur de tyrosinase de formule (II) :
R₅-Ile-Ser-Leu-Leu-Asp-Ala-Gln-Ser-R₆ R₅-[(SEQ ID NO: 2)-R₆] (II)
où
R₅ est choisi dans le groupe consistant en H-, R₇-C(O)- et R₇-OC(O)-;
R₆ est choisi dans le groupe consistant en -OH, -OR₇, -SR₇ et -NR₇R₈;
R₇ et R₈ sont choisis indépendamment parmi H-, C₁-C₂₄ alkyle, C₂-C₂₄ alcényle et C₆-C₁₀ aryle;
et les sels acceptables du point de vue cosmétique et solvates de ceux-ci.

4. Microcapsule et/ou nanocapsule selon l'une quelconque des revendications 1 à 3, **caractérisée en ce que** c'est une microcapsule ou nanocapsule polymérique, de préférence une microcapsule ou nanocapsule polymérique à deux couches, où les polymères formant la microcapsule et/ou nanocapsule polymérique sont choisis dans le groupe consistant en un poly(D,L-lactide-co-glycolide), les poly(acides lactiques), un copolymère séquencé poly(propylène fumarate-co-éthylèneglycol) [P(PF-co-EG)], un poly-anhydride poly-anhydride (fumarique-co-sébacique), un poly(oxyde d'éthylène)-poly(lactide/glycolide), un poly(acide aminé), un poly(alcool vinylique), un alginate, le dextrane, le chitosane, l'hydroxyapatite, le collagène, la fibrine, l'acide hyaluronique, les carbomères, un poly(acide aminé), le poly(éthylèneglycol).

5. Microcapsule ou nanocapsule selon la revendication 4, **caractérisée en ce que** la microcapsule et/ou nanocapsule polymérique à deux couches comprend un poly(D,L-lactide-co-glycolide) comme polymère de couche interne et un poly(alcool vinylique) comme polymère de couche externe.

6. Microcapsule ou nanocapsule selon l'une quelconque des revendications 1 à 5, **caractérisée en ce que** le composé de formule (I) est couplé de manière covalente au polymère de couche externe de la microcapsule.

7. Microcapsule ou nanocapsule selon l'une quelconque des revendications 1 à 6, **caractérisée en ce que** la distribution de dimensions de la microcapsule et/ou nanocapsule est de 10 à 10000 nm.

8. Procédé pour l'obtention des microcapsules et/ou nanocapsules comme défini dans les revendications 1 à 7 comprenant les étapes de
a) formation de la microcapsule ou nanocapsule encapsulant le composé blanchissant la peau et
b) couplage d'un composé de formule (I) :
R₂-Ser¹-Tyr²-Ser³-Nle⁴-Glu⁵-His⁶-DPhe⁷-Arg⁸-(AA₁)⁹-Gly¹⁰-Lys¹¹-DPro¹²-Val¹³-R₁ (I)
où
R₁ est choisi dans le groupe consistant en -OH, -OR₃, -SR₃, -NR₃R₄,-NHR₉, le polyéthylèneglycol ou un dérivé de celui-ci et -OR₉ où R₉ est le polyéthylèneglycol ou un dérivé de celui-ci ;
R₂ est choisi dans le groupe consistant en H-, R₃-C(O)- et R₃-OC(O)-,
R₃ et R₄ sont choisis indépendamment parmi H, C₁-C₂₄ alkyle, C₂-C₂₄ alcényle et C₆-C₁₀ aryle ;
AA₁ est choisi dans les groupes d'acides aminés qui représentent un groupe d'acides aminés aromatiques comme: le tryptophane, la 3-(2-naphtyl)-D-alanine, l'acide 3-amino-3-(1-naphtyl)-propionique, l'acide 3-amino-3-(biphényl)-propionique, la phénylalanine, la tyrosine, l'histidine, la thyrosine, le 5-hydroxy-tryptophane et la L-3,4-dihydroxyphénylalanine;
et les sels acceptables du point de vue cosmétique et solvates de ceux-ci,
au polymère de surface externe de la microcapsule ou nanocapsule avant ou après la formation de la microcapsule.

9. Procédé selon la revendication 8, où la microcapsule ou nanocapsule est une microcapsule ou nanocapsule à deux couches polymérique ayant un polymère de couche interne et un polymère de couche externe et l'étape a) de formation de la microcapsule ou nanocapsule comprend les étapes de
a1) mélange du polymère de couche interne avec le composé blanchissant la peau dans un solvant approprié,
a2) émulsification du mélange obtenu dans l'étape a) avec le polymère de couche externe dans un solvant approprié, et éventuellement
a3) isolement des microcapsules.

10. Composition cosmétique comprenant la microcapsule et/ou nanocapsule comme défini dans l'une quelconque des revendications 1 à 7.

11. Utilisation du peptide de formule (I) comme défini dans la revendication 1 pour l'obtention de microcapsules ou nanocapsules pour la prévention cosmétique et/ou le traitement cosmétique des taches brunes cutanées.

12. Utilisation d'une microcapsule ou nanocapsule comme défini dans l'une quelconque des revendications 1 à 7 pour la prévention cosmétique et/ou le traitement cosmétique des taches brunes cutanées.
